# EUROPEAN PATENT APPLICATION

(11) **EP 4 578 938 A1**
(43) Date of publication of application: **02.07.2025**
(21) Application number: 22960213.1
(22) Date of filing: 30.09.2022
(51) Int. Cl.: C12M 1/34, C12Q 1/68, G01N 33/50, C40B 20/02

(54) **BIOCHIP AND USE AND PREPARATION METHOD THEREFOR**

(71) Applicant: BGI Shenzhen, Shenzhen, Guangdong 518083 (CN)
(72) Inventor: HONG, Yan, Shenzhen, Guangdong 518083 (CN); LI, Quanshui, Shenzhen, Guangdong 518083 (CN); CHEN, Ao, Shenzhen, Guangdong 518083 (CN); LI, Yuxiang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Altmann Stößel Dick Patentanwälte PartG mbB
(86) International application number: PCT/CN2022/123027
(87) International publication number: WO 2024/065585

(57) **Abstract**

Provided are a biochip (100) and a method of using and preparing a biochip. The biochip (100) includes: a solid support having a front surface (101) and a back surface (102); a plurality of feature sites (110) arranged in an array and a plurality of bio-molecular sequences attached to the plurality of feature sites (110) formed on the front surface, each bio-molecular sequence includes at least a positioning sequence, the positioning sequence includes information indicating a spatial position of the bio-molecular sequence on the biochip (100); and at least one of a first marker (120) recognizable by human eyes and a second marker (130) recognizable by an instrument provided on the back surface.

## Description

### TECHNICAL FIELD

The embodiments of the present disclosure generally relate to the field of biotechnology, and more specifically, to a biochip with an identifiable marker and a method of using and preparing a biochip. The biochip may, for example, be used in a spatial omics or spatiotemporal omics analysis instrument, so as to perform marking, capturing, etc. of spatial omics in situ information.

### BACKGROUND

Spatial heterogeneity is a key feature of biological organ functions, and positional information of biological cells is very important for researches of cell fate regulation mechanisms and cell lineage generation processes. Therefore, it is required to simultaneously record transcriptional heterogeneity and spatial coordinates of biological cells in order to better understand the biological cells. Generally, a technique of quantifying transcriptional expression while simultaneously recording spatial coordinates is referred to as "spatial transcriptional omics sequencing technology". If sample information from different time periods is superimposed, such technique may provide researchers with information in both temporal and spatial dimensions, thus it may also be referred to as "spatiotemporal omics" technology.

Nucleic acid or gene sequencing (for example, including DNA sequencing and RNA sequencing) is one of the important methods of studying biological nucleic acids or genes. As a part of gene sequencing, the spatial omics technology involving spatial dimension or the spatiotemporal omics technology involving temporal and spatial dimensions uses the biochip technology to load probes with spatial position information onto chips. These chips capture target nucleic acids or proteins in biological tissues, and then convert the captured information or products into corresponding nucleotide sequences through biochemical reactions and recycle them. Finally, cDNA or RNA in the recycled sample is sequenced through second-generation sequencing, through a one-to-one correspondence between captured information and spatial coordinates, read contents are overlaid onto tissue images to generate complete gene expression images of tissue slices, providing a new research direction for cognitive organ structures, life developments, human diseases, and species evolutions. This technology has a high value in clinical research and cancer diagnosis.

When performing spatial omics or spatiotemporal omics processing or sequencing operations, it is often required to automatically or manually distinguish or identify different biochips, for example, for understanding biochemical reaction processes on various chips, establishing a correspondence or matching relationship between the captured products, sequenced data, and detected biological tissues. In practice, these operations generally involve a plurality of chips with similar appearances, and during manual or automated operations, there may be problems such as difficulty in distinguishing different chips and matching errors.

### SUMMARY

In order to solve at least one of the above-mentioned and other problems and deficiencies in the prior art, the present disclosure is provided.

According to an aspect of the present disclosure, a biochip is provided, including: a solid support having a front surface and a back surface; a plurality of feature sites arranged in an array and a plurality of bio-molecular sequences attached to the plurality of feature sites formed on the front surface, each bio-molecular sequence includes at least a positioning sequence, and the positioning sequence includes information indicating a spatial position of the bio-molecular sequence on the biochip; and at least one of a first marker recognizable by human eyes and a second marker recognizable by an instrument provided on the back surface.

In some embodiments, each bio-molecular sequence further includes a probe configured to capture a biological substance.

In some embodiments, the probe includes at least a capture sequence linked to the positioning sequence, and a biochemical reaction is performed on the probe and the biological substance to be captured.

In some embodiments, each feature site is attached with one bio-molecular sequence or a plurality of copied bio-molecular sequences, and positioning sequences of the copied bio-molecular sequences attached to one and the same feature site are the same.

In some embodiments, the bio-molecular sequence includes a nucleic acid molecule, such as a DNA nanoball.

In some embodiments, each bio-molecular sequence further includes a fixing sequence allowing an annealing and an initial extension reaction of a complementary nucleotide sequence of the fixing sequence.

In some embodiments, the nucleic acid molecule includes a unique molecular identifier (UMI) sequence, and the positioning sequence includes a coordinate identifier (CID) sequence.

In some embodiments, at least the positioning sequences of the bio-molecular sequences attached to different feature sites are different.

In some embodiments, a position of each feature site on the front surface is mapped one-to-one with the spatial position indicated by the positioning sequence of the bio-molecular sequence attached to the feature site.

In some embodiments, the feature site is defined by a well, a micro-pore, a protrusion, or a modified planar region formed on the front surface.

In some embodiments, a central distance between adjacent feature sites is within a range of 100 nm to 800 nm.

In some embodiments, the first marker and/or the second marker include unique identity recognition information for determining an identity of the biochip.

In some embodiments, the first marker includes a character number, and the second marker includes a QR code, a barcode, or an electronic tag.

In some embodiments, the first marker and the second marker are arranged side by side on the back surface.

In some embodiments, the biochip has a shape of rectangle with a size in a range of 0.5 cm×0.5 cm to 10 cm×10 cm, such as a size in a range of 1 cm×1 cm to 6 cm×6 cm.

In some embodiments, a material of the biochip is silicon, glass or polymer material.

According to another aspect of the present disclosure, a method of analyzing spatial omics information of a biological tissue sample is provided, including: placing the biological tissue sample on a front surface of the biochip of any one of claims 1 to 18, and loading the biochip into an analysis instrument; performing a biochemical reaction between the biological tissue sample and a plurality of biological molecular sequences of the biochip, so as to generate a reaction product including the positioning sequence; and analyzing the reaction product to restore spatial position information of a cell in the biological tissue sample based on a spatial position indicated by the positioning sequence.

In some embodiments, the method further includes: before loading the biochip into the analysis instrument, entering unique identity recognition information of the biochip into the analysis instrument based on the first marker and/or the second marker.

According to yet another aspect of the present disclosure, a method of preparing the biochip described in any embodiment of the present disclosure is provided, including: providing a substrate having a front surface and a back surface; forming at least one of the first marker and the second marker on the back surface of the substrate; forming a plurality of feature sites arranged in an array on the front surface of the substrate; and attaching a plurality of bio-molecular sequences to the plurality of feature sites in a physical or chemical method, each bio-molecular sequence includes at least a positioning sequence, and the positioning sequence includes information indicating a spatial position of the bio-molecular sequence on the biochip.

In some embodiments, the first marker or the second marker is formed on the back surface through at least one of etching, coating, pasting and printing.

In some embodiments, the substrate includes a silicon wafer, a glass plate or a polymer film sheet, and the method further includes: after attaching the plurality of bio-molecular sequences to the front surface, cutting the substrate into a plurality of chip units, each cut chip unit has at least one of the first marker and the second marker uniquely corresponding to the chip unit, and each cut chip unit has a predetermined size to serve as a single biochip.

Through the detailed descriptions of the present disclosure with reference to the accompanying drawings in the following, other purposes and advantages of the present disclosure will be apparent and may assist in a comprehensive understanding of the present disclosure.

### BRIEF DESCRIPTION OF THE DRAWINGS

The features and advantages of the present disclosure may be more clearly understood with reference to the accompanying drawings. The drawings are illustrative and should not be construed as limiting the present disclosure in any way, in which:
FIG. 1 is a back view schematically showing a substrate including a plurality of uncut chip units according to an exemplary embodiment of the present disclosure;
FIG. 2 is a front view schematically showing a biochip according to an exemplary embodiment of the present disclosure;
FIG. 3 is a back view schematically showing a biochip according to an exemplary embodiment of the present disclosure;
FIG. 4 is a partial enlarged perspective view schematically showing a structure of a part of a biochip according to an exemplary embodiment of the present disclosure;
FIG. 5 is a cross-sectional view schematically showing a structure of a biochip according to an exemplary embodiment of the present disclosure;
FIG. 6 is a block diagram schematically showing a method of analyzing spatial omics information of a biological tissue sample using a biochip according to an exemplary embodiment of the present disclosure; and
FIG. 7 is a block diagram schematically showing a method of preparing a biochip according to an exemplary embodiment of the present disclosure.

### DETAILED DESCRIPTION OF EMBODIMENTS

In the following, technical solutions in the embodiments of the present disclosure will be described clearly and completely in conjunction with the accompanying drawings. Apparently, the described embodiments are only part of the embodiments of the present disclosure, rather than all the embodiments. Based on the embodiments in the present disclosure, all other embodiments obtained by those of ordinary skill in the art without creative labor are within the scope of protection of the present disclosure.

In addition, in the following detailed descriptions, for ease of explanation, many specific details are elaborated to provide a comprehensive understanding of the embodiments of contents of the present disclosure. However, it is apparent that one or more embodiments may also be implemented without these specific details. In other cases, well-known structures and devices are illustrated to simplify the drawings.

Spatial omics technology (such as spatial genome, spatial transcriptome, spatial proteome, spatial immunome) use a biochip as a vector to convert information in a biological tissue into a detectable form through biochemical reactions, involving repeated rounds of manual or automated operations and a plurality of steps of biochemical reactions based on different principles. In these operations or different biochemical reactions, it is often required to distinguish or recognize different biochips, for example, for entering chip information, associating each chip with a spatial omics processing flow, determining a biochemical reaction progress on each chip, establishing a correspondence or matching relationship between captured products, sequenced data and the detected biological tissues, and so on.

According to the exemplary embodiments of the present disclosure, a biochip is provided, which may be used, for example, in a spatial omics or spatiotemporal omics processing instrument, so as to perform marking, capturing, etc. of spatial omics in situ information. As described below, the biochip provided according to the exemplary embodiments of the present disclosure has at least a marker that facilitates chip distinguishing or recognition, so as to more effectively improve accuracy and flux of biological detection.

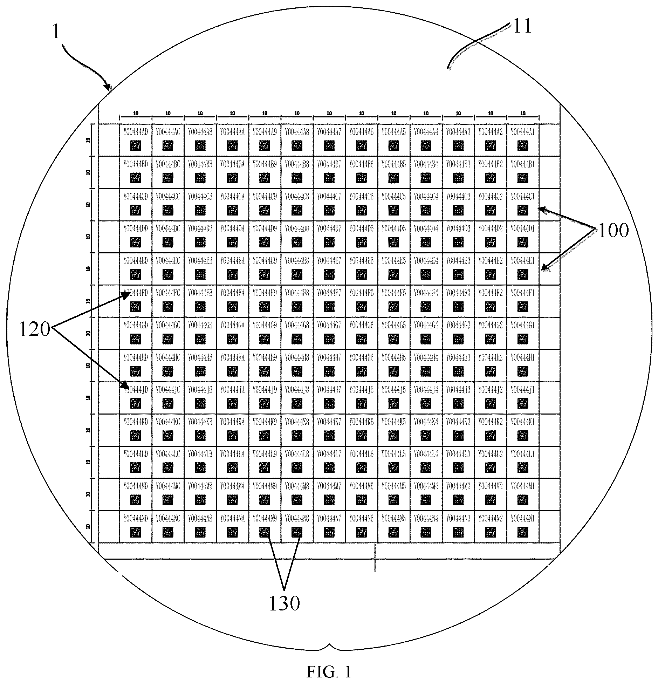

FIG. 1 schematically shows a back surface 11 of a substrate 1. The substrate 1 may be in a form of a wafer, including a plurality of chip units arranged in an array according to an exemplary embodiment of the present disclosure. A single chip unit herein is referred to as a biochip 100. The substrate 1 may include a silicon wafer, a glass plate, a polymer film sheet, and the like. Each chip unit may be cut from the substrate 1 to serve as the biochip 100 in a cutting manner, such as by manual tools, automated cutting apparatuses such as a laser cutting machine, and the like. It will be understood that, in some applications, the substrate itself with a plurality of chip units arranged in an array that have not been cut may also be referred to or may serve as the biochip.

A shape of the cut biochip is not limited, it may be quadrangular, such as rectangular or square, or it may be a chip with a non-standard size, such as having any size within a range of 0.5 cm×0.5 cm to 10 cm×10 cm, such as 0.5 cm×0.5 cm, 1 cm×1 cm, 1 cm×2 cm, 2 cm×3 cm, 3 cm×4 cm, 3 cm×5 cm, 4 cm×6 cm, 5 cm×6 cm, 6 cm×6 cm, 6 cm×7 cm, 7 cm×7 cm, 7 cm×8 cm, 8 cm×8 cm, 8 cm×9 cm, 9 cm×9 cm, 9 cm×10 cm, 10 cm×10 cm, and so on. As an example, a thickness T of the provided biochip may be in a range of 400 µm to 1000 µm, preferably 725 µm.

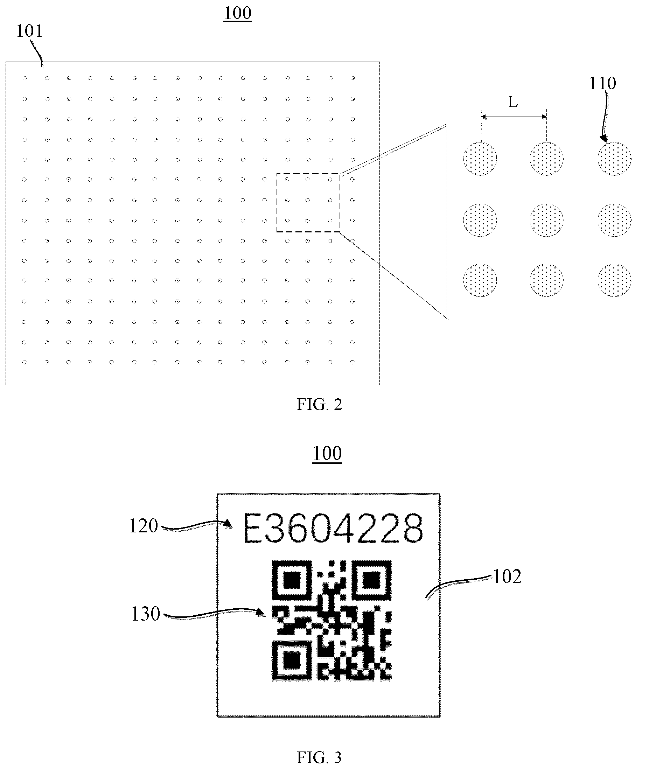

The biochip described herein includes a solid support or a vector, which may be an uncut substrate itself or a part cut from the substrate 1. The biochip has a front surface and a back surface opposite to the front surface. For example, as shown in FIG. 2 and FIG. 3, the biochip 100 has a front surface 101 and a back surface 102. It will be understood that the terms "front" and "back" described herein only refer to two opposite surfaces of the chip, and are not intended to limit these surfaces to have a specific orientation or direction. As shown in FIG. 2, a plurality of feature sites 110 arranged in an array and a plurality of bio-molecular sequences (not shown) attached (such as linked or fixed) to the plurality of feature sites 110 are formed on the front surface 101 of the biochip 100. Each bio-molecular sequence includes at least a positioning sequence, such as a barcode sequence. The positioning sequence includes information indicating a spatial position of the bio-molecular sequence on the biochip. As an example, the positioning sequence may include, for example, a coordinate identification (CID) sequence determined through sequencing. The CID corresponds one-to-one with a coordinate position of each bio-molecular sequence. By establishing a mapping relationship between the CID and the coordinate position, subsequent captured biological substances may be restored to its spatial position.

As an example, the biological molecule sequences carried on the biochip may include genes or nucleic acid molecules (such as DNA or RNA fragments), proteins, polypeptides, polysaccharide molecules, and the like. Specifically, the bio-molecular sequence may include a vector sequence attached to the feature site, and the positioning sequence may be a part of the vector sequence. In some examples, the vector sequence or the positioning sequence may include a nucleic acid sequence, such as a nucleotide sequence or a single-stranded DNA sequence. For example, a complementary sequence of the vector sequence may be subjected to an amplification reaction, such as rolling circle amplification (RCA) and PCR amplification, so as to obtain a vector sequence including a plurality of copies.

For example, in the spatial omics analysis, the bio-molecular sequence attached to the feature site 110 may be used to capture (such as recognize, bind or hybridize) a target cellular substance in the biological tissue, and may also be used, in subsequent reactions, for a connection between the vector sequence and a probe or a capture sequence for capturing, so that the captured substance or reaction product carries the positioning sequence or corresponding spatial position information. In an exemplary embodiment, each bio-molecular sequence may also include a probe for capturing the target biological substance, such as a probe cluster having one or more probes. The probe includes at least a capture sequence linking with the vector sequence, such as the positioning sequence. The biochemical reaction may be performed between the capture sequence and the biological substance to be captured, so as to capture a cellular substance in the biological tissue, such as capturing target nucleic acids or proteins, such as mRNA, in the biological tissue slice placed on the front surface of the chip. The capture sequence may hybridize with all or part of the nucleic acid to be captured, for example, the capture sequence may include oligonucleotide sequences capable of capturing mRNA, or random or degenerate oligonucleotide sequences, or specific sequences for specific target nucleic acids.

In some embodiments, the vector sequence on the cut biochip may also include a fixing sequence (also referred to as a "linking sequence"). The fixing sequence allows annealing and an initial extension reaction of a complementary nucleotide sequence of the fixing sequence. Each bio-molecular sequence may also include a first nucleic acid molecule and a second nucleic acid molecule. The first nucleic acid molecule has a complementary sequence to hybridize with the fixing sequence and the positioning sequence of the vector sequence, so as to form a double strand. The second nucleic acid molecule is linked to the first nucleic acid molecule (via a ligase) and may include a capture sequence used as a part of a probe. In other embodiments, the fixing sequence may be pre-attached to the chip. During subsequent use, the nucleic acid molecule with the capture sequence may be linked to the fixing sequence through suitable biochemical reactions, and then the chip may be used to capture the biological substance.

In an exemplary embodiment, each feature site 110 may be attached to or held with one bio-molecular sequence or more copied bio-molecular sequences, and the positioning sequences of the copied bio-molecular sequences attached to or held by the same feature site are the same, so that it may be determined that the substance captured by the probe at one and the same feature site has the same spatial position information corresponding to the position of the feature site. On the other hand, the bio-molecular sequences attached to different feature sites have at least different positioning sequences, so as to distinguish and position the spatial position of the captured biological substance. Further, the position of each feature site 110 on the front surface of the chip is in one-to-one mapping relationship or correspondence to the spatial position indicated by the positioning sequence of the bio-molecular sequence attached to the feature site, thereby restoring the subsequent captured biological substance (such as mRNA or cDNA) to their spatial positions. For example, omics information derived from a biological tissue or cell sample to be analyzed may be mapped or associated with an image spatial site of the tissue sample according to the positional information, thereby obtaining spatial omics information of the tissue sample.

In a preferred embodiment, the bio-molecular sequence attached to the feature site may include a DNA nanoball (DNB) and a probe linked to the DNB. The DNB is, for example, a product obtained after rolling circle amplification by taking a single-stranded circular DNA as a template. The DNB fixed on the chip may be sequenced to obtain CID information, which corresponds one-to-one with a coordinate position of the DNB attached to each feature site. By establishing a mapping relationship between the CID and the coordinate position of the DNB, the subsequent mRNA captured by the probe attached to the DNB may be restored to its spatial position. For example, each probe or the nucleic acid molecule attached to (such as hybridized with a vector sequence) a feature site may include or be linked with a unique molecular identifier (UMI) sequence (e.g., for distinguishing different nucleic acid molecules or transcripts), and/or a polyT sequence including oligonucleotides, or other fragments or sequences for recognizing and combining with mRNA or cDNA, thereby capturing free mRNA. Subsequently, the captured product or information is converted into a corresponding nucleotide sequence through biochemical reactions, and being recycled. Finally, the cDNA or RNA in the recycled sample is sequenced through second-generation sequencing, the captured information is in one-to-one correspondence with the spatial coordinates, and the read content is superimposed on the tissue image, thereby generating a complete gene expression image of the tissue slice.

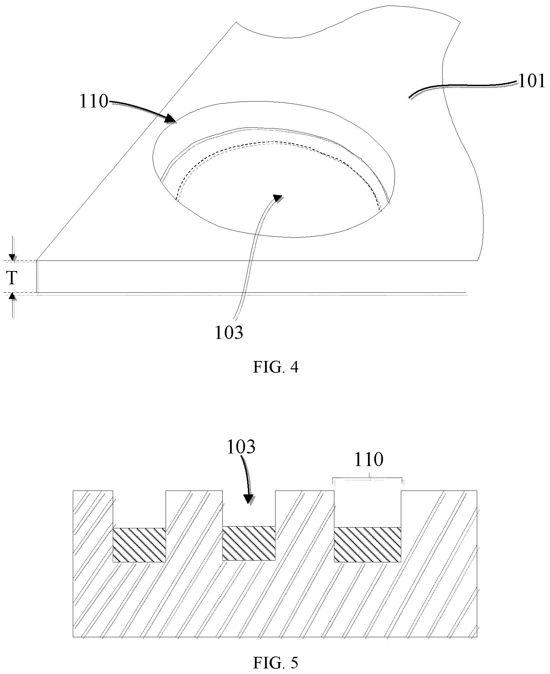

The feature site 110 formed on the chip may be defined by a discrete well 103 (for example, with reference to FIG. 4 and FIG. 5), a micro-pore, a micro-well, a protrusion, a micro-bead, or a modified planar region formed on the front surface 101 of the chip, which is generally set to have a size such as length, width, or possibly depth or height suitable for attaching the aforementioned bio-molecular sequence. For example, the feature sites 110 may be arranged in an array on the front surface 101, and a central distance L between adjacent feature sites 110 may be in a range of 50 nm to 900 nm, preferably in a range of 100 nm to 800 nm, such as 900 nm, 800 nm, 750 nm, 600 nm, 500 nm, even 300 nm, 100 nm or 50 nm. As an example, a diameter of the DNB may be 220 nm or less, and a central distance between two adjacent DNBs may be 500 nm or less, thus may achieve spatial transcriptional omics technology with high resolution.

According to an exemplary embodiment of the present disclosure, the biochip 100 also has a marker for chip distinguishing or recognition. This recognition marker is unique or specific to each biochip to distinguish one from another, so that different chips may be distinguished or recognized based on such markers, so as to enter chip information, associate each chip with spatial omics processing flows, determine biochemical reaction processes on each chip, establish a correspondence or matching relationship between the captured products, sequenced data, and detected biological tissues.

In the embodiments shown in FIG. 1 and FIG. 3, such recognition markers are provided or formed on the back surface 102 of the biochip 100. In other embodiments, in a case that a size of the front surface of the biochip is large enough or allowed, such recognition markers may also be formed on the front surface of the chip to facilitate recognition operations. For example, such markers may be formed on the chip through etching, coating, pasting, silkscreening, printing, and other methods. Apparently, such recognition markers do not participate in or interfere with the subsequent biochemical reactions that will be performed on the front surface of the chip.

As an example, as shown in FIG. 1 and FIG. 3, such recognition markers formed on the biochip 100 may include at least one of a first marker 120 recognizable by human eyes and a second marker 130 recognizable by an instrument. The formed first marker 120 and/or second marker 130 are uniquely corresponding to the biochip, that is, the first marker 120 and/or second marker 130 of one biochip are different from the first markers 120 and/or second markers 130 of other biochips. As an example, the first marker 120 and/or the second marker 130 may include identity recognition information for determining an identity of the biochip 100. Such identity recognition information is unique or distinct for each biochip, used to distinguish different chips.

For example, the first marker may be formed to facilitate distinction and recognition of chips by human eyes during manual operation, while the second marker may be recognized by a spatial omics or spatiotemporal omics processing instrument, such as being read by a scanner for system entering or being associated with a chip container, or being used on an instrument for automatic scanning and entering information, binding with a processing flow of the instrument, etc., including: recognizing the chips being processed or to be processed and/or their biochemical reaction processes and results during operations, such as cleaning, extracting waste liquid, adding sealing liquid, adding antibody drops and incubation, permeabilization, reverse transcription, tissue removal, cDNArelease, product recycle, staining, imaging, etc., or quickly and easily finding and identifying chips of interest from the plurality of chips.

In the embodiments of the present disclosure, the specific forms of the first marker 120 and the second marker 130 formed on the biochip 100 are not limited, and various suitable markers or marker combinations that may be recognized by human eyes/instruments may be used, including but not be limited to optical markers, electrical markers, magnetic markers, and the like. As an example, the first marker 120 may include a character number, such as a human eye-recognizable letter and/or a number code, a text, a pattern, or a combination thereof, and the second marker 130 may include a QR code, a barcode, or an electronic tag (such as an RF tag), etc., and a letter, a number, a text or a pattern that may be recognized or read by the instrument may also be used.

In the embodiments shown in FIG. 1 and FIG. 3, both the first marker 120 and the second marker 130 are arranged side by side on the back surface 102 of the biochip 100, so as to facilitate accurate recognition of the chip during both manual operation and automated instrument operation. In this case, the first marker is preferably different from the second marker, which may be different in type or size, for example, same in type but different in size, or same in size but different in type, or different in both type and size. It will be understood that in some examples, the first marker for human eye recognition may be designed to be unrecognizable by the instrument, so as to avoid an occurrence of instrument misrecognition.

In some examples, an entire size of the first marker 120 and the second marker 130 does not exceed a surface area of the back surface of the chip, such as not exceeding 10 mm × 10 mm. For example, a total length of the first marker 120 may not exceed 10 mm, and a width of the first marker 120 may not be less than 1 mm but not greater than 10 mm; and/or, a length of the second marker 130 and a width of the second marker 130 may not be less than 1 µm, respectively. The first marker 120 or the second marker 130 may be a marker engraved into the back surface 102, and an engraving depth may be, for example, in a range of 3 µm to 10µm, preferably 5µm.

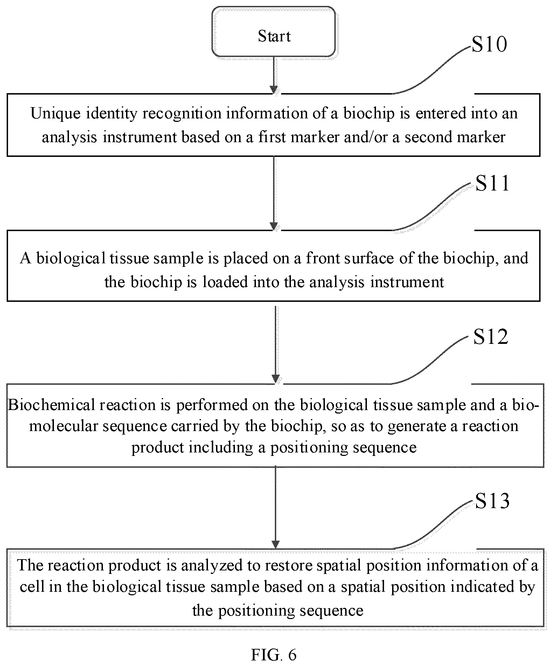

The biochip provided according to the embodiments of the present disclosure may be used in spatial omics or spatiotemporal omics analysis or sequencing instruments, so as to perform spatial omics in situ information marking, analysis, capturing, and so on. In an exemplary embodiment, as shown in FIG. 6, a method of capturing, analyzing or acquiring spatial omics information of a biological tissue sample is provided, mainly including step S11 to step S13.

In step S11, a biological tissue sample or slice is placed on the front surface of the biochip described in any embodiment of the present disclosure, and the biochip is loaded into an analysis instrument.

In step S12, a biochemical reaction is performed on a cellular substance in the biological tissue sample or slice and a bio-molecular sequence carried by the biochip, so as to generate a captured substance or a reaction product including a positioning sequence. For example, a frozen slice of a fresh tissue is loaded onto a surface of the chip, then fixation and permeabilization are performed, and finally reverse transcription and amplification are performed.

In step S13, the reaction product is analyzed to restore spatial position information of a cell in the biological tissue sample based on a spatial position indicated by the positioning sequence. For example, an amplified cDNA may be collected as a template for preparing a library and may be sequenced together with the CID. By calculating and analyzing sequenced data, spatially resolved transcriptomic research may be achieved.

The obtained spatial position information may be used for analysis or sequencing of spatial omics (such as spatial transcriptome, spatial proteome, etc.), and may be used to mark the position information of the cellular substance (such as RNA, DNA, protein, etc.) in the biological tissue or which cell does the cellular substance comes from, so as to study spatial specificity of the substance. For example, the captured product or information may be converted into a corresponding nucleotide sequence through biochemical reactions, and may be recycled, such as collecting the amplified cDNA, and sequencing the cDNA or RNA in the recycled sample through, for example, second-generation sequencing technology. The captured information may be in one-to-one correspondence with the spatial position information, and the read content may be superimposed on the captured image of the tissue, thereby generating a complete gene expression image of the tissue slice, such as constructing a tissue in situ spatial transcription map, which may in situ indicate new cell clusters, cell-cell interactions. After the spatial-temporal transcriptome information is in correspondence with clinical images, a relationship between pathological phenotypes and molecular information may be further studied, revealing the cellular heterogeneity and transcriptome changes of human tissues at high resolution.

Before loading the biochip into the analysis instrument, the method may further include step S10: unique identity recognition information of a biochip is entered into an analysis instrument based on a first marker 120 and/or a second marker 130, such as read by a scanner for system entering or associating with a chip container, or automatic scanning and entering information on the instrument, so that each chip may be associated or bound with spatial omics processing flows to determine biochemical reaction processes on each chip (including but not be limited to operations such as cleaning, extracting waste liquid, adding sealing liquid, adding antibody drop and incubation, permeabilization, reverse transcription, tissue removal, cDNA release, product recycle, staining, imaging, etc.), a correspondence or matching relationship between the captured products, the sequenced data and the detected biological tissues is established, or chips of interest may be quickly and easily found and identified from a plurality of chips.

It will be recognized that the biochip provided according to the embodiments of the present disclosure may be applied not only to the capture of spatial omics, but also to omics markers and pathology markers, including but not be limited to in situ fluorescence staining, conventional pathological staining, and the like.

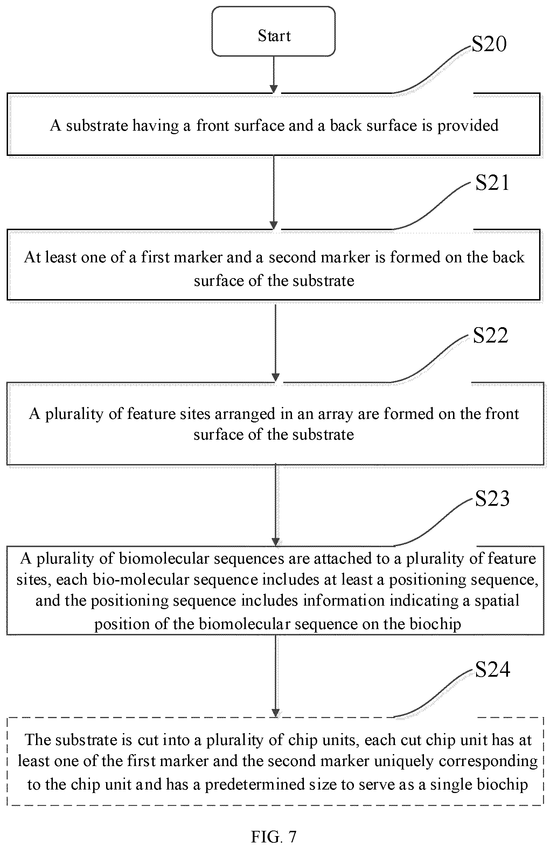

The exemplary embodiments according to the present disclosure further provide a method of preparing a biochip, as shown in FIG. 1 and FIG. 7, which mainly includes steps as follow.

In step S20, a substrate 1 having a front surface and a back surface 11 is provided. The substrate includes, for example, a silicon wafer, a glass plate, a polymer film sheet or the like.

In step S21, at least one of a first marker 120 and a second marker 130 is formed on the back surface 11 of the substrate 1. For example, the first marker 120 or the second marker 130 may be formed on the back surface of the substrate through etching, coating, pasting, silkscreening, or printing, etc.

In step S22, a plurality of feature sites 110, such as a well 103, a micro-pore, a micro-well, a protrusion, a micro-bead, or a modified planar region, arranged in an array are formed on the front surface of the substrate 1, for example, through physical, chemical or biological methods.

In step S23, a plurality of bio-molecular sequences are attached to the plurality of feature sites 110, for example, through physical or chemical method or biochemical reactions. Each bio-molecular sequence includes at least a positioning sequence, and the positioning sequence includes information indicating a spatial position of the bio-molecular sequence on the biochip.

In some embodiments, the substrate to which the bio-molecular sequence is attached may be cut into smaller chips for different applications. For example, with reference to FIG. 7, the method of preparing a biochip may further include step S24: after the plurality of bio-molecular sequences are attached to the front surface of the substrate, the substrate is cut into a plurality of chip units by, for example, manual tools, automated cutting apparatuses such as laser cutting machines, and the like. Each cut chip unit has at least one of the first marker and second marker that uniquely correspond to the chip unit, and each cut chip unit has a predetermined size to serve as a single biochip.

Although the embodiments of the present disclosure have been shown and described, it will be understood by those skilled in the art that changes may be made to these embodiments without departing from principles and spirit of the present disclosure, and the scope of the present disclosure is defined by the appended claims and their equivalents.

## Claims

1. A biochip, comprising:
a solid support having a front surface and a back surface;
a plurality of feature sites (110) arranged in an array and a plurality of bio-molecular sequences attached to the plurality of feature sites formed on the front surface, wherein each bio-molecular sequence comprises at least a positioning sequence, and the positioning sequence comprises information indicating a spatial position of the bio-molecular sequence on the biochip; and
at least one of a first marker (120) recognizable by human eyes and a second marker (130) recognizable by an instrument provided on the back surface.

2. The biochip of claim 1, wherein each bio-molecular sequence further comprises a probe configured to capture a biological substance.

3. The biochip of claim 2, wherein the probe comprises at least a capture sequence linked to the positioning sequence, and a biochemical reaction is performed on the probe and the biological substance to be captured.

4. The biochip of claim 1, wherein each feature site (110) is attached with one bio-molecular sequence or a plurality of copied bio-molecular sequences, and positioning sequences of the copied bio-molecular sequences attached to one and the same feature site are the same.

5. The biochip of claim 4, wherein the bio-molecular sequence comprises a nucleic acid molecule.

6. The biochip of claim 5, wherein the nucleic acid molecule comprises a DNA nanoball.

7. The biochip of claim 5, wherein each bio-molecular sequence further comprises a fixing sequence allowing an annealing and an initial extension reaction of a complementary nucleotide sequence of the fixing sequence.

8. The biochip of claim 5, wherein the nucleic acid molecule comprises a unique molecular identifier (UMI) sequence, and the positioning sequence comprises a coordinate identifier (CID) sequence.

9. The biochip of claim 1, wherein at least the positioning sequences of the bio-molecular sequences attached to different feature sites are different.

10. The biochip of claim 9, wherein a position of each feature site (110) on the front surface is mapped one-to-one with the spatial position indicated by the positioning sequence of the bio-molecular sequence attached to the feature site.

11. The biochip of any one of claims 1 to 10, wherein the feature site is defined by a well (103), a micro-pore, a protrusion, or a modified planar region formed on the front surface.

12. The biochip of any one of claims 1 to 10, wherein a central distance (L) between adjacent feature sites (110) is within a range of 100 nm to 800 nm.

13. The biochip of any one of claims 1 to 10, wherein the first marker (120) and/or the second marker (130) comprise unique identity recognition information for determining an identity of the biochip.

14. The biochip of any one of claims 1 to 10, wherein the first marker (120) comprises a character number, and the second marker (130) comprises a QR code, a barcode, or an electronic tag.

15. The biochip of any one of claims 1 to 10, wherein the first marker (120) and the second marker (130) are arranged side by side on the back surface (102).

16. The biochip of any one of claims 1 to 10, wherein the biochip has a shape of rectangle with a size in a range of 0.5 cm×0.5 cm to 10 cm×10 cm.

17. The biochip of claim 16, wherein the biochip has a shape of rectangle with a size in a range of 1 cm×1 cm to 6 cm×6 cm.

18. The biochip of any one of claims 1 to 10, wherein a material of the biochip is silicon, glass or polymer material.

19. A method of analyzing spatial omics information of a biological tissue sample, comprising:
placing the biological tissue sample on a front surface of the biochip of any one of claims 1 to 18, and loading the biochip into an analysis instrument;
performing a biochemical reaction between the biological tissue sample and a plurality of biological molecular sequences of the biochip, so as to generate a reaction product comprising the positioning sequence; and
analyzing the reaction product to restore spatial position information of a cell in the biological tissue sample based on a spatial position indicated by the positioning sequence.

20. The method of claim 19, further comprising: before loading the biochip into the analysis instrument,
entering unique identity recognition information of the biochip into the analysis instrument based on the first marker (120) and/or the second marker (130).

21. A method of preparing the biochip of any one of claims 1 to 18, comprising:
providing a substrate having a front surface and a back surface;
forming at least one of the first marker (120) and the second marker (130) on the back surface of the substrate;
forming a plurality of feature sites (110) arranged in an array on the front surface of the substrate; and
attaching a plurality of bio-molecular sequences to the plurality of feature sites, wherein each bio-molecular sequence comprises at least a positioning sequence, and the positioning sequence comprises information indicating a spatial position of the bio-molecular sequence on the biochip.

22. The method of claim 21, wherein the first marker or the second marker is formed on the back surface through at least one of etching, coating, pasting and printing.

23. The method of claim 21 or 22, wherein the substrate comprises a silicon wafer, a glass plate or a polymer film sheet, and the method further comprises:
after attaching the plurality of bio-molecular sequences to the front surface, cutting the substrate into a plurality of chip units, wherein each cut chip unit has at least one of the first marker and the second marker uniquely corresponding to the chip unit, and each cut chip unit has a predetermined size to serve as a single biochip.
